# EUROPEAN PATENT APPLICATION

(11) **EP 3 738 554 A1**
(43) Date of publication of application: **18.11.2020**
(21) Application number: 19174572.8
(22) Date of filing: 15.05.2019
(51) Int. Cl.: A61F 2/915, A61F 2/06, A61F 2/856, A61F 2/82

(54) **STENT FOR IMPLANTATION INTO A BLOOD VESSEL OF A PATIENT**

(71) Applicant: Müller, Roman, 76133 Karlsruhe (DE)
(72) Inventor: Müller, Roman, 76133 Karlsruhe (DE)
(74) Representative: Durm Patentanwälte PartG mbB

(57) **Abstract**

The present invention relates to a stent (10) for implantation into a blood vessel of a patient in a connection area of the blood vessel and another blood vessel of the patient, the stent extending along a longitudinal axis (16), comprising: a first stent section (12) for extending into the blood vessel, said first stent section having a substantially tubular shape; and a second stent section (14) for being positioned in the connection area of the blood vessel and the other blood vessel of the patient, said second stent section being connected to the first stent section at a proximal end of the second stent section, wherein a diameter (D) of the stent in the second stent section orthogonal to the longitudinal axis increases in a direction of a distal end of the second stent section; and a plane (P) defining the distal end of the second stent section is inclined with respect to the longitudinal axis at an inclination angle (a) different from a right angle. The present invention further relates to a method for manufacturing a stent.

## Description

The present invention relates to a stent for implantation into a blood vessel of a patient in a connection area of said blood vessel and another blood vessel of the patient. The present invention further relates to a method for manufacturing a stent for implantation into a blood vessel of a patient in a connection area of said blood vessel and another blood vessel of the patient.

Annually, between one and two out of a thousand people aged from twenty to forty years experience a venous thrombosis. At an age of over seventy-five years this number increases to ten out of a thousand people. In this respect a deep vein thrombosis (DVT) relates to the formation of a blood clot in a deep vein, most commonly the legs. Symptoms of DVT include swollen, heavy legs and constant pain. DVT can severely restrict the quality of life of those affected.

A related condition that may cause DVT and result in comparable symptoms is the May-Thurner-Syndrome (MTS), also known as the iliac vein compression syndrome. MTS is a condition in which compression of the common venous outflow track of the left lower extremity may cause discomfort, swelling, pain or blood clots in the iliofemoral veins.

One option for treating DVT and MTS and the corresponding symptoms is the use of a stent in the iliac vein in the area of the bifurcation, i.e. the meeting point of the two iliac veins with the inferior vena cava. Stents for being used in this respect have to meet the specific anatomical requirements of this application area. In particular, a stent has to provide a sufficient chronic outward force and radial resistance force against external force effects caused by leg movements of the patient. Since current stents are mainly designed for arterial use, they are often not adapted to the requirements of a venous application and the specific anatomical situation in the connection area of the iliac vein and the inferior vena cava.

In this respect, WO 2017/012673 A1 relates to a stent for transluminal implantation in hollow organs, in particular in blood vessels, ureters, esophagi, colons, duodena, or bile ducts. The stent comprises a substantially tubular body which can be transferred from a compressed state having a first cross-sectional diameter into an expanded state having an enlarged second cross-sectional diameter. The stent comprises a plurality of cells which are defined by bordering elements formed by the tubular body. The stent is distinguished in that some of the cells are extended in the longitudinal direction of the stent in comparison with the remaining cells in order to form a slanted end phase of the stent. This proposed design, however, still has drawbacks with respect to finding the optimal position of the stent upon implantation. Further, possible complications can result from the stent slipping out of place due to external force effects.

In view of the above, it is an object of the present invention to provide a stent that is suitable for implantation into a blood vessel of a patient in a connection area of said blood vessel with another blood vessel. It is desired to provide a stent that does not slip and that provides a suitable alignment of the blood vessel to the other blood vessel. In particular, it is desired to provide a stent for being used in the connection area of the iliac vein and the inferior vena cava. The stent should preferably be able to align to different angles of the iliac bifurcation and prevent a slipping/gliding of the (right) iliac artery to the inferior vena cava.

To solve this problem, an aspect of the present invention relates to a stent for implantation into a blood vessel of a patient in a connection area of said blood vessel and another blood vessel of the patient, the stent extending along a longitudinal axis, comprising:
a first stent section for extending into the blood vessel, said first stent section having a substantially tubular shape; and
a second stent section for being positioned in the connection area of the blood vessel and the other blood vessel of the patient, said second stent section being connected to the first stent section at a proximal end of the second stent section, wherein
a diameter of the stent in the second stent section orthogonal to the longitudinal axis increases in a direction of a distal end of the second stent section; and
a plane defining the distal end of the second stent section is inclined with respect to the longitudinal axis at an inclination angle different from a right angle.

In another aspect, the present invention relates to a method for manufacturing a stent for implantation into a blood vessel of a patient in a connection area of said blood vessel and another blood vessel of the patient, the stent extending along a longitudinal axis and comprising a first stent section for extending into the blood vessel, said first stent section having a substantially tubular shape, and a second stent section for being positioned in the connection area of the blood vessel and the other blood vessel of the patient, said second stent section being connected to the first stent section at a proximal end of the second stent section. The method comprises the steps of:
using a tubular green body with a diameter in a second green body section corresponding to the second stent section orthogonal to a longitudinal axis of the green body increasing in a direction of a distal end of the second green body section; and
cutting the second green body section in a plane defining the distal end, said plane being inclined with respect to the longitudinal axis at an inclination angle different from a right angle.

Preferred embodiments of the invention are defined in the dependent claims. It shall be understood that the claimed method has similar and/or identical preferred embodiments as the claimed stent, in particular as defined in the dependent claims as disclosed herein.

In comparison to previous approaches, the stent of the present invention has a distal end that is inclined with respect to the longitudinal axis, i.e. oblique, and has an increasing diameter in the direction of the distal end of the stent, i.e. is flared. The stent thus has a flared and oblique section at its distal end.

This flared oblique section allows for a good alignment of one blood vessel (in particular the iliac vein) to another blood vessel (in particular the inferior vena cava) on the one hand and, on the other hand, allows for a good alignment of different angles of the bifurcation (in particular the iliac bifurcation). In addition, slipping/gliding of the blood vessel to the other blood vessel is prevented. The proposed stent design allows following the natural track of the blood vessel without straightening the blood vessel. In case the patient moves (walking, sitting, stairclimbing etc.), the proposed design allows a long application time and decreases risks of complications at a later stage. Further, the stent can be implanted into the blood vessel in the connection area of said blood vessel and the other blood vessel and can adapt to different angles of the bifurcation that might be present for different patients. Consequently, a cost-efficient manufacturing is possible since a single design can adapt to different angles and is suitable for different patients.

In a preferred embodiment, the inclination angle of the plane defining the distal end of the second stent section is between thirty degrees and sixty degrees, in particular between thirty-five degrees and fifty-five degrees. This inclination angle provides a good alignment of the stent in the connection area of the iliac vein and the inferior vena cava for most patients. It becomes possible to accommodate most anatomical situations.

In an embodiment, the increasing diameter in the second stent section is increasing in the direction of the distal end of the second stent section. In other words, a function describing the increase of the diameter has a positive second derivative. This allows accommodating the anatomical condition at the meeting of the iliac vein with the inferior vena cava. A slipping of the stent is prevented. The stent adapts to the patient's anatomy.

In a preferred embodiment, a change in diameter of the stent in the first stent section and in the second stent section is continuous. There are no kinks in the design of the stent. By making use of a continuously shaped stent it becomes possible to avoid complications due to obstructions of the flow of blood. The stent design is adapted to the natural shape of the vein. A good implantability is assured.

In a preferred embodiment, the first stent section includes at least two rings of rhombuses for providing a chronic outward force and a radial resistance force. The rhombuses of a ring are connected to one another at opposite rhombus vertices representing connection vertices. Further, the at least two rings are connected to one another via a number of axial ring connectors, said number being lower than a number of rhombuses in one ring of rhombuses. The stent includes multiple rings of rhombuses that are connected by means of axial ring connectors. This diamond ring design provides a high chronic outward force and a high radial resistance force to assure that the stent expands the blood vessel and prevents the blood vessel from collapsing. In addition, this design allows expanding and compressing the stent during implantation. Furthermore, this design makes it possible to cope with movements, i.e. provides a flexibility of the stent with respect to movements. This is particularly important since the stent is applied in the iliac vein in the connection area of the iliac vein and the inferior vena cava where frequent bending of the stent can occur when the patient is moving. The connection of the rings by means of the axial ring connectors results in a low axial bending force to follow the natural track of the vessel and allow for movement during application. Further, the risk of fatigue fracture is lowered since the bending stress is distributed. In comparison to previous stent designs this has the advantage that complications are avoided.

In a preferred embodiment, the axial ring connectors connect the at least two rings of rhombuses from a connection vertex of a first ring of rhombuses to a connection vertex of a second ring of rhombuses. By connecting the rhombuses from valley to valley instead of from peak to peak, a maximum length of the axial connectors can be obtained albeit assuring that the original function of the stent of expanding and stabilizing the blood vessel is maintained. A keel over of the diamond rings is made possible. The stent can follow the natural track and cope with movements by exerting a low axial bending force. Further, the risk of a fracture is decreased since the bending stress is distributed over a longer section of the stent.

In another preferred embodiment, an axial ring connector includes two connection sections for connecting to the connection vertices and a middle section for connecting the connection sections. The connection sections extend parallel to the longitudinal axis of the stent and the middle section is inclined versus the longitudinal axis of the stent. By this design, it becomes possible to align the rhombuses of one ring with the rhombuses of the neighboring ring to minimize the area in which the blood vessel is not stabilized. The original functionality of the stent is maintained and long axial connectors can be used resulting in the above-mentioned advantages.

In a preferred embodiment, the number of axial connectors connecting two neighboring rings is three. The use of three axial connectors for connecting two neighboring rings of rhombuses provides an optimal trade-off between stability and low axial bending force. In particular, when a constant material thickness is used in the production of the stent, the use of three axial connectors provides a good relation between the bending force and the exerted chronic outward force and radial resistance force of the stent.

In another preferred embodiment, the second stent section includes a plurality of rhombuses for providing a chronic outward force and a radial resistance force. The rhombuses of the plurality of rhombuses are connected to one another at rhombus edges representing connection edges. By connecting the rhombuses at their edges in the second stent section, it becomes possible to obtain a higher resistance and exert a higher force in the second stent section in comparison to the first stent section. The externally induced movement is mainly located in the iliac vein. In the area connecting the iliac vein to the inferior vena cava, less movement is induced if the patient moves his legs. Thus, designing a second stent section that is stiffer than the first stent section results in an adequate balancing. The connection area of the inferior vena cava and the iliac vein is reliably expanded.

In a preferred embodiment, the second stent section and the first stent section are connected via axial section connectors that preferably correspond to the axial ring connectors. Further, said axial section connectors connect connection vertices of the first stent section to vertices of connection edges of the second stent section. The axial section connectors allow a robust connection of the two stent sections. Again, a maximum possible length of the section connectors is obtained by connecting from valley to valley of the rhombuses. A high flexibility of the stent is obtained.

Preferably, the stent includes an oval termination element for terminating the second stent section at its distal end. The oval termination element is preferably elliptical. The oval termination element is connected to the second stent section and extends in the plane defining the distal end of the second stent section. The termination element thereby reduces the risk of injuries, in particular when the patient moves and the stent might change its position versus the blood vessel.

Preferably, the stent is formed in one piece. For instance, the stent may be manufactured by making use of a tubular green body and by later cutting this green body. This allows manufacturing a robust stent that is resistant against material fatigue.

Preferably, the stent is manufactured from nitinol.

The method of the present invention may particularly be used for manufacturing the stent of the present invention.

Herein, a tubular shape particularly corresponds to an elongated cylindrical shape. The definition of the terms proximal and distal is to be understood from a user's point of view. In particular, a distal end of a stent faces away from the surgeon implanting the stent.. A proximal end of a stent faces in the direction of the surgeon. This definition avoids ambiguities and unclarities that could result from whether the surgery is carried out above or below the patient's heart and whether the stent is implanted into an artery or a vein. A rhombus can also be referred to as a diamond. A ring of rhombuses is a ring structure wherein multiple rings constitute the stent, in particular in the first stent section. Neighboring rings means rings that are located next to one another. The stent of the present invention can be compressed and expanded again after insertion into the blood vessel (implantation).

The stent (and the method) of the present invention is generally suitable for being used in arteries and veins (blood vessels) in any connection area of two blood vessels. However, one particular beneficial application area is the use at the connection are of the iliac vein (corresponding to the blood vessel) and the inferior vena cava (corresponding to the other blood vessel). This area is usually referred to as the (iliac) bifurcation.

These and other aspects of the invention will be apparent from and elucidated with reference to the embodiment(s) described hereinafter. In the following drawings
- Figure 1: shows a schematic illustration of the stent for implantation into the iliac vein of a patient according to the present invention;
- Figure 2: shows a schematic illustration of the stent in the area of the connection of the first stent section to the second stent section; and
- Figure 3: schematically illustrates a method for manufacturing a stent according to the present invention.

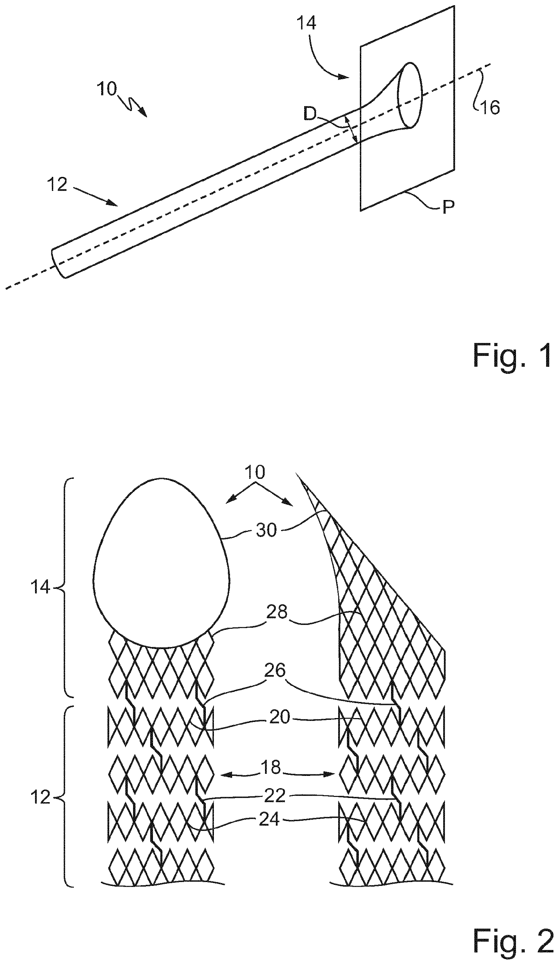

Fig. 1 shows a schematic illustration of a stent 10 according to the present invention. In the illustrated example, the stent 10 is especially intended for the treatment of MTS and DVT and suitable for implantation into an iliac vein of a patient (corresponding to the blood vessel) in the area of the connection (bifurcation) of the iliac vein and the inferior vena cava (corresponding to the other blood vessel). The requirements of a stent to be applied in this area is that the constant angles of the vein have to be followed without straightening the vein. Further, the movement inside the pelvis during ambulation (walking, sitting, stairclimbing etc.) have to be followed. The risk of a bending kink during movement has to be reduced. The stent has to withstand a flat compression from the ligaments in the area of the pelvis. Further, it has to withstand local compression from the iliac artery. Thus, a dedicated venous stent has to assure that the vessel is not straightened since this could lead to a kink of the vein at the end of the stented area. The movement has to be followed during ambulation and a long lifetime has to be assured without material fatigue failure in spite of the movement of the vessel. The stent has to withstand and restore ongoing flat and local compression and avoid occlusion.

The stent 10 of the present invention is comprised of a first stent section 12 and a second stent section 14 and substantially extends along a longitudinal axis 16. In the first stent section 12 the stent 10 has a substantially tubular shape and is suitable for being inserted into the iliac vein. In the second stent section 14, the stent 10 has a flared and oblique shape for being positioned in the connection area of the iliac vein and the inferior vena cava.

A diameter D of the stent 10 in the second stent section 14 increases in the direction of the distal end of the second stent section 14, i.e. in the direction of the inferior vena cava and away from the first stent section 12. This results in the flaring of the stent and allows a reliable positioning and a resistance against a slipping of the stent 10. In the illustrated example, the increase in diameter D in the second stent section increases in the direction of the distal end of the second stent section 14. This shape allows the stent 10 to remain at its position and to accommodate different angles of the bifurcation for different patients. Thus, it becomes possible to manufacture a stent that is suitable for being used for a variety of anatomical shapes of different patients. Furthermore, the change in diameter D of the entire stent 10 for both stent sections 12, 14 is continuous so that the stent has no kinks that could lead to complications due to obstructions of the blood flow or due to problems upon implantation.

A plane P defining the distal end of the second stent section 14 is inclined with respect to the longitudinal axis 16 at an inclination angle a that is different from a right angle. This oblique design makes it possible that the stent 10 is placed at the bifurcation and follows the natural track of the blood vessel in this area. The inclination angle a is preferably about 50°, which allows accommodating the angle at the bifurcation for most patients.

In Fig. 2 an embodiment of the stent 10 of the present invention is schematically illustrated in its distal part. The left illustration shows a frontal view of the stent 10 in the direction of the distal opening. The illustration on the right side of Fig. 2 shows a corresponding side view.

As illustrated, the first stent section 12 is substantially comprised of multiple rings 18 of rhombuses 20 that are connected to one another via axial ring connectors 22. The individual rhombuses 20 of one ring are connected to one another at connection vertices 24. The connection vertices 24 of one particular rhombus 20 are opposite vertices of said rhombus 20. The rhombus structure allows compressing the stent 10 for inserting it into the vein and expanding it again after insertion. The rhombuses 20 can also be referred to as diamonds and the rings 18 can also be referred to as diamond rings.

As further illustrated in Fig. 2, the axial ring connectors 22 connect the different rings 18 from valley to valley, i.e. from one connection vertex 24 of one ring 18 to another connection vertex 24 of a neighboring ring 18. By making use of a connection from valley to valley instead of a connection from peak to peak, a maximum length of the axial connectors 22 is obtained. This allows reducing an axial bending force that is required for bending the stent 10. Thereby, it is assured that the stent 10 can follow the natural track of the blood vessel and can cope with movements of the blood vessel, in particular in the first stent section 12. Preferably, three axial connectors 24 are used for connecting two neighboring rings 18.

The axial ring connectors 24 are preferably comprised of three sections as illustrated in Fig. 2. Two connection sections that extend parallel to the longitudinal axis of the stent are connected to one another via a middle section that is inclined versus the longitudinal axis of the stent. This design allows obtaining an overlapping arrangement of the rhombuses 20 of neighboring rings 18. In particular, the peaks of the rhombuses 20 of one ring 18 are aligned with the valleys of rhombuses 20 of the neighboring ring 18 as described above. A good support of the blood vessel can be obtained albeit providing a flexibility with respect to movements of the blood vessel or the patient, respectively.

At the connection between the first stent section 12 and the second stent section 14, axial section connectors 26 are used. In the second stent section 14, the rhombuses 20 are connected to one another at their edges. This has the effect that the stent 10 has a lower flexibility in its second stent section 14 than in its first stent section 12. This is advantageous since the movement of the vein in the area of the connection of the iliac vein to the inferior vena cava is reduced in comparison to the movement of the lower iliac vein. The connection edges 28 provide a stable and robust design.

As further illustrated in Fig. 2, the stent 10 includes an oval termination element 30 that terminates the second stent section 14 at its distal end. This oval termination element 30 is a solid portion of the stent that connects the vertices of the rhombuses of the distal end of the second stent section 14. This oval termination element 30 is preferably elliptical and basically has the form of an ellipse that is situated in the plane that defines the distal end of the second stent section 14. It is to be understood that this oval termination element 30 is optional and that the stent can also be put to use without the oval termination element 30. In particular, it is possible to make use of the rhombus structure in the second stent section 14. The plane that defines the distal end of the second stent section 14 then includes vertices of the rhombuses.

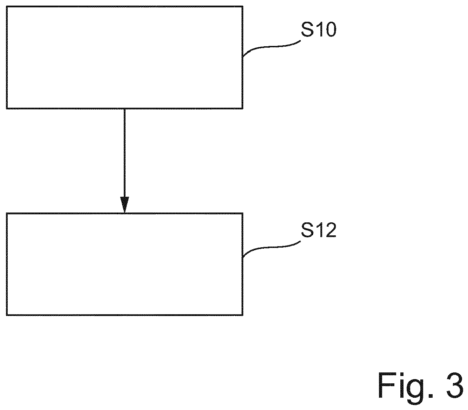

In Fig. 3, a method for manufacturing a stent for implantation into an iliac vein is schematically illustrated. The method includes steps of using S10 a tubular green body and cutting S12 the second green body section. The method may be used for controlling a laser cutting tool or another cutting tool upon manufacturing the stent. For this, the method may be implemented in software.

The foregoing discussion discloses and describes merely exemplary embodiments of the present disclosure. As will be understood by those skilled in the art, the present disclosure may be embodied in other specific forms without departing from the spirit or essential characteristics thereof. Accordingly, the description is intended to be illustrative, but not limiting the scope of the disclosure, as well as other claims. The disclosure, including any readily discernible variants of the teachings herein, defines, in part, the scope of the foregoing claim terminology such that no inventive subject matter is dedicated to the public.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

In so far as embodiments of the disclosure have been described as being implemented, at least in part, by software-controlled data processing apparatus, it will be appreciated that a non-transitory machine-readable medium carrying such software, such as an optical disk, a magnetic disk, semiconductor memory or the like, is also considered to represent an embodiment of the present disclosure. Further, such software may also be distributed in other forms, such as via the Internet or other wired or wireless telecommunication systems. A method according to the present invention may particularly be carried out to control the operation of a software defined radio.

### Reference numerals

- 10: stent
- 12: first stent section
- 14: second stent section
- 16: longitudinal axis
- 18: ring of rhombuses
- 20: rhombus
- 22: axial ring connector
- 24: connection vertex
- 26: axial section connector
- 28: connection edge
- 30: oval termination element
- D: diameter
- P: plane
- a: inclination angle

## Claims

1. Stent (10) for implantation into a blood vessel of a patient in a connection area of said blood vessel and another blood vessel of the patient, the stent extending along a longitudinal axis (16), comprising:
a first stent section (12) for extending into the blood vessel, said first stent section having a substantially tubular shape; and
a second stent section (14) for being positioned in the connection area of the blood vessel and the other blood vessel of the patient, said second stent section being connected to the first stent section at a proximal end of the second stent section, wherein
a diameter (D) of the stent in the second stent section orthogonal to the longitudinal axis increases in a direction of a distal end of the second stent section; and
a plane (P) defining the distal end of the second stent section is inclined with respect to the longitudinal axis at an inclination angle (a) different from a right angle.

2. Stent (10) according to claim 1, wherein the inclination angle (a) of the plane (P) defining the distal end of the second stent section (14) is between 30° and 60°, in particular between 35° and 55°.

3. Stent (10) according to any one of the preceding claims, wherein the increase in diameter (D) in the second stent section (14) is increasing in the direction of the distal end of the second stent section.

4. Stent (10) according to any one of the preceding claims, wherein a change in diameter (D) of the stent in the first stent section (12) and in the second section is continuous.

5. Stent (10) according to any one of the preceding claims, wherein
the first stent section (12) includes at least two rings (18) of rhombuses (20) for providing a chronic outward force and a radial resistance force, wherein
the rhombuses of a ring are connected to one another at opposite rhombus vertices (24) representing connection vertices; and
the at least two rings are connected to one another via a number of axial ring connectors (22), said number being lower than a number of rhombuses in one ring of rhombuses.

6. Stent (10) according to claim 5, wherein the axial ring connectors (22) connect the at least two rings (18) of rhombuses (20) from a connection vertex (24) of a first ring of rhombuses to a connection vertex of a second ring of rhombuses.

7. Stent (10) according to any one of claims 5 and 6, wherein
an axial ring connector (22) includes two connection sections for connecting to the connection vertices and a middle section for connecting the connection sections; and
said connection sections extend parallel to the longitudinal axis (16) of the stent and said middle section is inclined versus the longitudinal axis of the stent.

8. Stent (10) according to any one of claims 5 to 7, wherein the number of axial connectors (22) connecting two neighboring rings (18) is three.

9. Stent (10) according to any one of the preceding claims, wherein
the second stent section (14) includes a plurality of rhombuses (20) for providing a chronic outward force and a radial resistance force; and
said plurality of rhombuses are connected to one another at rhombus edges representing connection edges (28).

10. Stent (10) according to claims 5 and 9, wherein
the second stent section (14) and the first stent section (12) are connected via axial section connectors (26) that preferably correspond to the axial ring connectors (22); and
said axial section connectors connect connection vertices (24) of the first stent section to vertices of connection edges of the second stent section.

11. Stent (10) according to any one of the preceding claims including
an oval termination element (30) for terminating the second stent section (14) at its distal end, wherein
said oval connection element is connected to the second stent section and extends in the plane (P) defining the distal end of the second stent section.

12. Stent (10) according to any one of the preceding claims, wherein the stent is formed in one piece.

13. Stent (10) according to any one of the preceding claims, wherein the stent is manufactured from nitinol.

14. Method for manufacturing a stent (10) for implantation into a blood vessel of a patient in a connection area of said blood vessel and another blood vessel of the patient, the stent extending along a longitudinal axis (16) and comprising a first stent section (12) for extending into the blood vessel, said first stent section having a substantially tubular shape, and a second stent section (14) for being positioned in the connection area of the blood vessel and the other blood vessel of the patient, said second stent section being connected to the first stent section at a proximal end of the second stent section, the method comprising steps of:
using (S10) a tubular green body with a diameter (D) in a second green body section corresponding to the second stent section orthogonal to a longitudinal axis of the green body increasing in a direction of a distal end of the second green body section; and
cutting (S12) the second green body section in a plane (P) defining the distal end, said plane being inclined with respect to the longitudinal axis at an inclination angle (a) different from a right angle.
